# EUROPEAN PATENT APPLICATION

(11) **EP 4 410 965 A1**
(43) Date of publication of application: **07.08.2024**
(21) Application number: 22873026.3
(22) Date of filing: 26.09.2022
(51) Int. Cl.: C12N 5/0783, A61K 35/17, A61P 29/00, A61P 37/02, A61P 37/06, A61P 37/08, C12N 5/0735, C12N 5/10, C12N 15/13

(54) **METHOD FOR PRODUCING T CELL**

(30) Priority: 27.09.2021 JP 2021157187
(71) Applicant: Kyoto University, Kyoto-shi, Kyoto 606-8501 (JP); Takeda Pharmaceutical Company Limited, Osaka-shi, Osaka 541-0045 (JP)
(72) Inventor: KANEKO, Shin, Kyoto-shi, Kyoto 606-8501 (JP); IRIGUCHI, Shoichi, Kyoto-shi, Kyoto 606-8501 (JP); KASSAI, Yoshiaki, Fujisawa-shi, Kanagawa 251-0012 (JP); SEKIYA, Keiko, Fujisawa-shi, Kanagawa 251-0012 (JP); MATSUDA, Atsushi, Fujisawa-shi, Kanagawa 251-0012 (JP); SATO, Takayuki, Fujisawa-shi, Kanagawa 251-0012 (JP)
(74) Representative: Jones, Nicholas Andrew
(86) International application number: PCT/JP2022/035626
(87) International publication number: WO 2023/048275

(57) **Abstract**

Disclosed is a method for producing regulatory T cells, the method comprising:
(1) differentiating cells that can differentiate into regulatory T cells, into which an expression construct is introduced, into regulatory T cells, the expression construct comprising:
(a) conserved non-coding sequence (CNS) 1, CNS2, and CNS3 of Foxp3 gene;
(b) a promoter; and
(c) a nucleic acid encoding FOXP3.

Also disclosed are regulatory T cells obtained by the method, and a pharmaceutical composition comprising the regulatory T cells.

## Description

### Technical Field

The present invention relates to a method for producing regulatory T cells, regulatory T cells obtained by the method, a pharmaceutical composition comprising the regulatory T cells, and the like. The present invention also relates to iPS cells into which an expression construct is introduced.

### Background of Invention

In recent years, due to the immune response suppression function of regulatory T cells (Treg), research and development of cell therapy using regulatory T cells (Treg therapy) have been promoted worldwide for the purpose of treating various immune disorders. Thus, regulatory T cells are expected to be used in the treatment of graft-versus-host disease (GvHD) and autoimmune diseases, and in the treatment and prevention of inflammatory diseases and allergic diseases.

Regulatory T cells are conveniently broadly divided into naturally occurring regulatory T cells and inducible regulatory T cells. Naturally occurring regulatory T cells are defined as being naturally produced in the thymus. Inducible regulatory T cells are defined as being differentiated from peripheral naive T cells in response to an external stimulus in an artificial tolerance induction model. These regulatory T cells are further subdivided according to the type of marker expressed in the cells.

When CD4⁺CD25⁺ regulatory T cells, which are one of the naturally occurring regulatory T cell group, are removed from a living body, various organ-specific autoimmune diseases develop spontaneously. At that time, transplantation of CD4⁺CD25⁺ regulatory T cells prevents the development of autoimmune diseases. Accordingly, naturally occurring CD4⁺CD25⁺ regulatory T cells were considered to play an important role in maintaining peripheral immune self-tolerance. Since then, it has become clear that CD4⁺CD25⁺ regulatory T cells can suppress not only autoimmunity but also most immune responses, such as inflammation due to foreign antigens, rejection due to transplantation, infectious immunity, allergies, and tumor immunity. At present, the transcription factor FOXP3 has been clarified as a master regulator of CD4⁺CD25⁺ regulatory T cells.

NPL 1 discloses that for immune dysregulation, polyendocrinopathy, enteropathy, X-linked (IPEX) syndrome caused by mutations in FOXP3, genes were introduced into autologous hematopoietic stem cells using a lentiviral vector to restore FOXP3 expression, and that the cells were administered to model mice with IPEX syndrome. As a result, the hematopoietic stem cells reportedly differentiated into functional regulatory T cells, resulting in recovery from the autoimmune phenotype. Further, PTL 1 discloses a similar recombinant lentiviral vector comprising a nucleotide sequence encoding a human FOXP3 protein.

PTL 2 discloses that expression of Mcl-1 together with Foxp3 is important for improving the survival of Treg. In addition, PTL 3 discloses a genetically engineered mammalian cell comprising a transgene encoding a lineage commitment factor that promotes differentiation into CD4⁺ Treg. PTL 4 discloses a method for preparing a composition of T cells from stem cells or progenitor cells by culturing a three-dimensional cell aggregate comprising a selected population of stromal cells expressing a Notch ligand and a selected population of stem cells or progenitor cells.

However, although industrial production methods with high production efficiency (yield) are desired for regulatory T cells, no reports have been made on methods for efficiently producing regulatory T cells.

FOXP3 is a master regulator of Treg. It is known that FOXP3 is constitutively expressed in Treg, and that constitutive expression is not observed in conventional T cells (Tconv, also referred to as effector T cells or inflammatory T cells) that do not have suppressive function. Therefore, studies on the maintenance of FOXP3 expression in primary Treg and the induction of FOXP3 expression in primary Tconv or bulk CD4 single-positive (SP) T cells have been conducted, and compounds having the activity of maintaining and inducing FOXP3 expression, cytokines, and combinations thereof have been reported. Such compounds, cytokines, and combinations thereof include AS2863619 as a CDK8/19 inhibitor (NPL 2), rapamycin as an mTOR inhibitor (NPL 3, NPL 4, NPL 5, and NPL 6), TNFR2 agonist antibody (NPL 5, NPL 7, and NPL 8), cytokine TGF-β (NPL 2 and NPL 6), and the like.

### Citation List

### Patent Literature

PTL 1: WO2019/040655
PTL 2: WO2014/180943
PTL 3: WO2021/092581
PTL 4: WO2017/075389

### Non-patent Literature

NPL 1: Cell Stem Cell 24, 309-317, February 7, 2019
NPL 2: Sci. Immunol. 4, eaaw2707 (2019)
NPL 3: Nat. Immunol. 20(9), 1208-1219 (2019)
NPL 4: Clin. Exp. Immunol. 197(1): 52-63 (2019)
NPL 5: Front. Immunol. 9:573, (2018)
NPL 6: PLoS One. 11(2), e0148474 (2016)
NPL 7: PLoS One. 11(5), e0156311 (2016)
NPL 8: Sci. Signal. 13, eaba9600 (2020)

### Summary of Invention

### Technical Problem

An object of the present invention is to provide a method for producing regulatory T cells that can produce regulatory T cells with high efficiency.

### Solution to Problem

As a result of extensive studies to achieve the above object, the present inventors found that regulatory T cells can be produced with high efficiency by introducing an expression construct comprising (a) CNS1, CNS2, and CNS3 of Foxp3 gene, (b) a promoter, and (c) cDNA of FOXP3, into cells that can differentiate into regulatory T cells (in particular, iPS cells), and inducing the differentiation of the obtained cells into regulatory T cells.

The present invention has been completed upon further examination based on this finding. The present invention provides a method for producing regulatory T cells, iPS cells, regulatory T cells, pharmaceutical composition, and the like described below.
[1] A method for producing regulatory T cells, the method comprising:
   (1) differentiating cells that can differentiate into regulatory T cells, into which an expression construct is introduced, into regulatory T cells, the expression construct comprising:
      (a) conserved non-coding sequence (CNS) 1, CNS2, and CNS3 of Foxp3 gene;
      (b) a promoter; and
      (c) a nucleic acid encoding FOXP3.
[2] The method according to [1], wherein the cells that can differentiate into regulatory T cells are pluripotent stem cells, hematopoietic stem cells, hemogenic endothelial cells, progenitor T cells, or mesodermal progenitor cells.
[3] The method according to [1] or [2], wherein the cells that can differentiate into regulatory T cells are iPS cells.
[4] The method according to any one of [1] to [3], wherein CNS1, CNS2, and CNS3 are located upstream of the promoter.
[4a] The method according to any one of [1] to [4], wherein the promoter is Foxp3 gene promoter.
[5] The method according to any one of [1] to [4a], wherein the regulatory T cells are CD25⁺/FOXP3⁺ cells.
[5a] The method according to any one of [1] to [5], wherein the regulatory T cells are CD25⁺/FOXP3⁺/CD4⁺ cells.
[6] The method according to any one of [1] to [5a], further comprising:
   (2) introducing an expression construct into cells that can differentiate into regulatory T cells, the expression construct comprising:
   (a) CNS1, CNS2, and CNS3 of Foxp3 gene;
   (b) a promoter; and
   (c) a nucleic acid encoding FOXP3.
[7] The method according to any one of [1] to [6], wherein a three-dimensional cell aggregate is cultured in step (1), and the three-dimensional cell aggregate comprises the cells that can differentiate into regulatory T cells or cells differentiated therefrom, and stromal cells expressing a Notch ligand.
[8] The method according to [7], wherein the cells that can differentiate into regulatory T cells or cells differentiated therefrom are CD34⁺ cells.
[9] The method according to [7] or [8], wherein the cells that can differentiate into regulatory T cells or cells differentiated therefrom are hemogenic endothelial cells.
[10] The method according to any one of [7] to [9], wherein the Notch ligand is at least one member selected from the group consisting of DLL4, DLL1, JAG1, and JAG2.
[11] iPS cells into which an expression construct is introduced, the expression construct comprising:
   (a) CNS1, CNS2, and CNS3 of Foxp3 gene;
   (b) a promoter; and
   (c) a nucleic acid encoding FOXP3.
[12] The iPS cells according to [11], wherein CNS1, CNS2, and CNS3 are located upstream of the promoter.
[13] Regulatory T cells obtained by the method according to any one of [1] to [10].
[14] A pharmaceutical composition comprising the regulatory T cells according to [13].
[15] The pharmaceutical composition according to [14], for use in prevention and/or treatment of an abnormally enhanced immune response.
[16] A method for preventing and/or treating an abnormally enhanced immune response, comprising administering the regulatory T cells according to [13] to a subject in need thereof.
[17] The regulatory T cells according to [13], for use in prevention and/or treatment of an abnormally enhanced immune response.
[18] Use of the regulatory T cells according to [13] in the production of a pharmaceutical composition for use in prevention and/or treatment of an abnormally enhanced immune response.
[19] The method according to any one of [1] to [10], further comprising:
   (3) culturing the regulatory T cells in the presence of an mTOR inhibitor and a TNFR2 agonist.
[19a] The method according to [19], wherein the mTOR inhibitor is rapamycin.
[19b] The method according to [19] or [19a], wherein the TNFR2 agonist is a TNFR2 agonist antibody.

### Advantageous Effects of Invention

The present invention makes it possible to produce regulatory T cells with high efficiency.

### Brief Description of Drawings

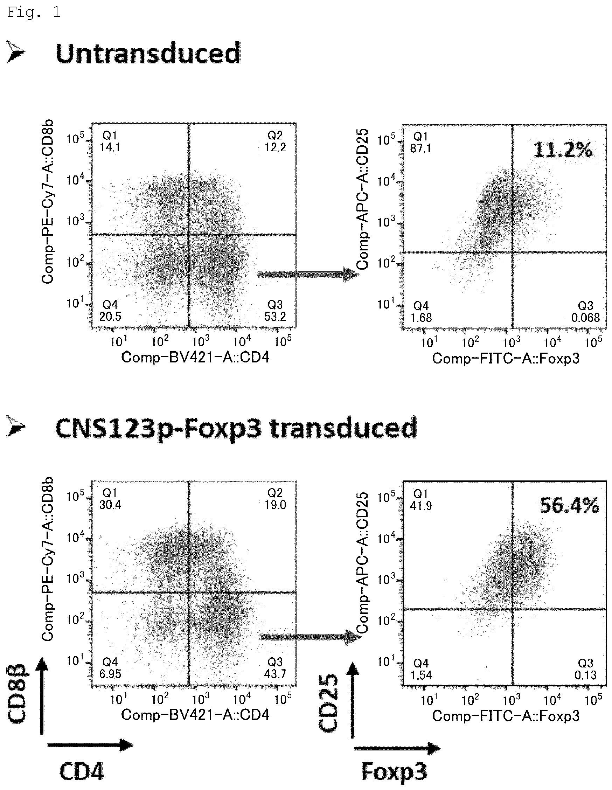

Fig. 1 shows the results of flow cytometry analysis after differentiation induction from iPS cells into Treg. Upper graphs: an expression construct-untransduced cell population as a control; lower graphs: a CNS-Foxp3 construct-transduced cell population. The numerical values in the graphs indicate the ratio of Treg (CD25⁺/FOXP3⁺) in each cell population.

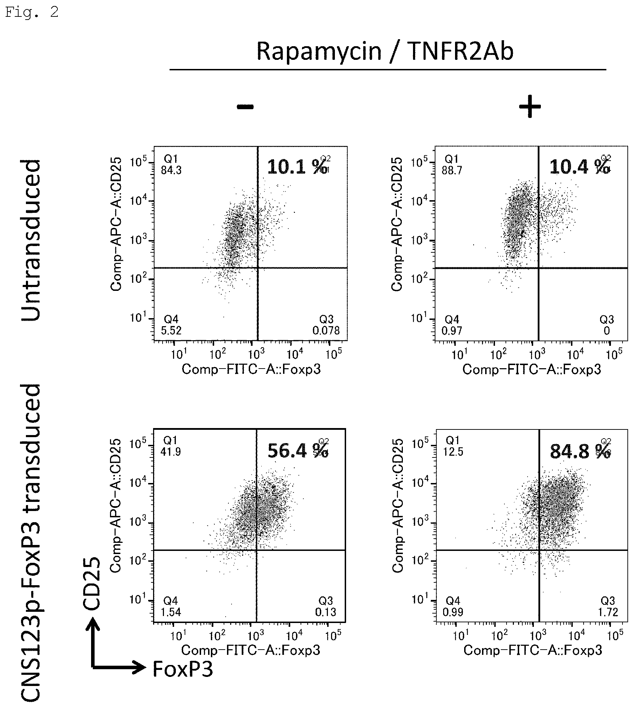

Fig. 2 shows the results of flow cytometry analysis of cells after differentiation from iPS cells into Treg after expansion culture in a medium containing rapamycin and anti-TNFR2 antibody. Upper graphs: an expression construct-untransduced cell population as a control; lower graphs: a CNS-Foxp3 construct-transduced cell population; left graphs: a cell population cultured in a medium not containing rapamycin and TNFR2Ab as a control; right graphs: a cell population cultured in a medium containing rapamycin and TNFR2Ab. The numerical values in the graphs indicate the ratio of Treg (CD3⁺/CD4⁺/CD8⁻/CD25⁺/Foxp3⁺) in each cell population.

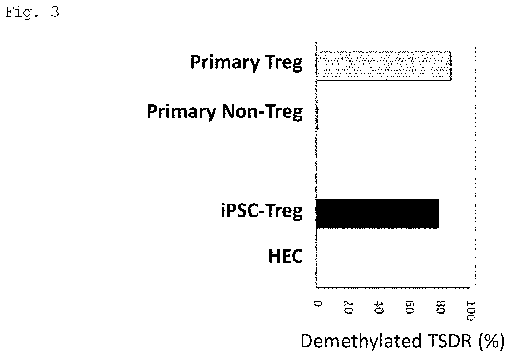

Fig. 3 is a graph showing the demethylation rate of TSDR (Treg-specific demethylated region) in cells after differentiation from iPS cells into Treg. Primary Treg: a Treg (CD3⁺/CD4⁺/CD8⁻/CD25⁺/CD127⁻) cell population isolated from human PBMC; Primary Non-Treg: a non-Treg (CD3⁺/CD4⁺/CD8⁻/CD25⁻) cell population isolated from human PBMC; iPSC-Treg: a Treg (CD3⁺/CD4⁺/CD8⁻/CD25⁺/Foxp3⁺) cell population differentiated from iPS cells; HEC: undifferentiated hematopoietic cells (CD34⁺/CD43⁻ /CD73⁻/CD184⁻) induced from iPS cells.

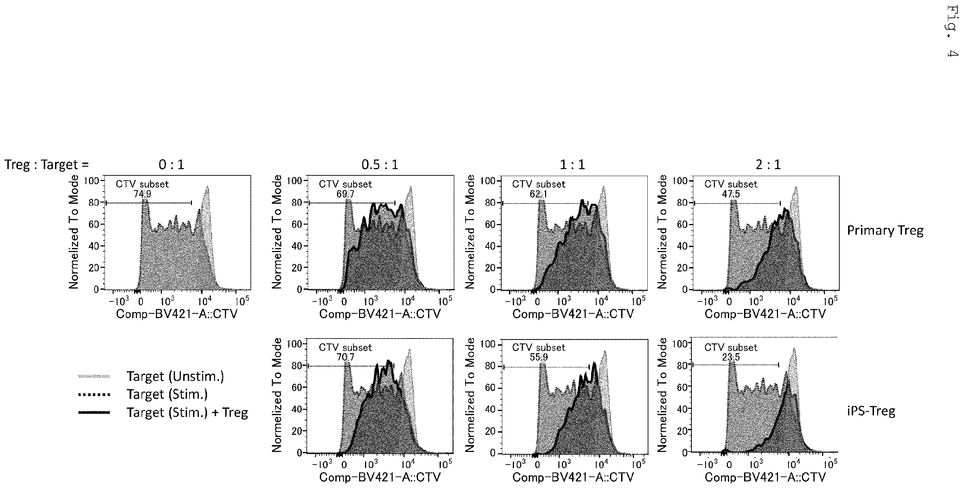

Fig. 4 shows the results of flow cytometry analysis after co-culture with human PBMC-derived allogeneic T cells using Treg differentiated from iPS cells. Upper graphs: a Treg (CD3⁺/CD4⁺/CD8⁻/CD25⁺/CD127⁻) cell population isolated from human PBMC; lower graphs: a Treg (CD3⁺/CD4⁺/CD8⁻/CD25⁺/Foxp3⁺) cell population differentiated from iPS cells. Treg:Target indicates the cell number ratio of Treg and human PBMC.

### Description of Embodiments

Embodiments of the present invention are described in detail below.

The term "comprise(s)" or "comprising" means that although elements following these terms are included, the inclusion is not limited to the elements. Therefore, these terms suggest inclusion of elements following them, but do not suggest exclusion of any other elements. The term "consist(s) of" or "consisting of" means that any elements following these terms are included, and that the inclusion is limited to the elements. Therefore, the term "consist(s) of" or "consisting of" indicates that the listed elements are required or essential, and that there are substantially no other elements. The term "consist(s) essentially of" or "consisting essentially of" means that any elements following these terms are included, and that there is a limitation to other elements that do not affect the activity or action specified in the present disclosure for the above elements. Therefore, the term "consist(s) essentially of" or "consisting essentially of" indicates that the listed elements are required or essential, while other elements are optional, and may be or may not be present depending on whether they affect the activity or action of the listed elements.

In the present specification, the term "culture" refers to maintenance or/and proliferation of cells in an in-vitro environment. The term "culturing" refers to maintaining or/and proliferating cells outside tissue or outside the body (e.g., in a cell culture dish or flask).

In the present specification, the phrase "culturing cells in the presence of a substance" refers to, for example, culturing the cells in a medium containing the substance. Examples of such culture include culture in a medium containing the substance alone or in a medium containing the substance, other differentiation-inducing factors, and the like. When the substance is added to the medium, it may be added directly to the medium, or may be dissolved in an appropriate solvent before use and then added to the medium. Further, culture can be performed while the substance is immobilized on a substrate or carrier surface during culture.

In the present specification, the term "positive (+)" means that a protein or gene is expressed in detectable amounts by methods known in the art. In the case of a protein that is expressed intracellularly and does not appear on the cell surface (e.g., a transcription factor or a subunit thereof), a reporter protein is expressed together with the protein, and the reporter protein is detected, whereby the target protein can be detected. Gene detection can be performed by using nucleic acid amplification and/or nucleic acid detection methods, such as RT-PCR, microarray, biochip, and RNAseq.

In the present specification, the term "negative (-)" means that the expression level of a protein or gene is below the lower limit of detection by all or any of the known methods described above. The lower limit of detection of protein or gene expression may vary depending on the method.

In the present specification, the term "marker" refers to a protein or its gene that is specifically expressed on the cell surface, in the cytoplasm, or in the nucleus of a given cell type. The marker is preferably a "cell surface marker." The "cell surface marker" refers to a protein expressed on the cell surface that can be labeled (stained) with fluorescent substances and that facilitates the detection, condensation, isolation, or the like of cells expressing the cell surface marker. The cell surface marker refers to a gene that is expressed (positive marker) or not expressed (negative marker) specifically in a given cell type, and specifically a substance that is produced (positive marker) or not produced (negative marker) as mRNA by transcription of the gene in the genome or as a protein by translation of the mRNA.

Such cell surface markers can be detected by immunological assays using antibodies specific for the cell surface markers, such as ELISA, immunostaining, and flow cytometry.

In the present specification, the term "expression" is defined as the transcription and/or translation of a specific nucleotide sequence driven by an intracellular promoter.

In the present specification, "pluripotent stem cells" refer to embryonic stem cells (ES cells) and cells with similar pluripotency, i.e. cells with the potential to differentiate into various tissues (endoderm, mesoderm, and ectoderm) of a living body. Examples of cells with the same pluripotency as that of ES cells include induced pluripotent stem cells (also referred to as "iPS cells" in the present specification).

In the present specification, "hematopoietic stem cells (HSC)" are multipotent stem cells that can differentiate into blood cells. Hematopoietic stem cells are mainly present in the bone marrow in a human living body, and differentiate into white blood cells (neutrophils, eosinophils, basophils, lymphocytes, monocytes, and macrophages), red blood cells, platelets, mast cells, dendritic cells, and the like. In the present specification, hematopoietic stem cells (HSC) can be positive to CD34 and negative to CD7 (CD34⁺/CD7⁻). In the present specification, "/" used in the phrase "CD34⁺/CD7⁻" etc. means "and."

In the present specification, "hemogenic endothelial cells (HEC)" are cells that express CD34 but do not express CD43, CD184, and CD73 (CD34⁺/CD43⁻/CD184⁻/CD73⁻) (CD34⁺/CD43⁻/CD184⁻/CD73⁻ cells do not express CD7, and are thus also referred to as "CD34⁺/CD7⁻/CD43⁻/CD184⁻/CD73⁻ cells") .

In the present specification, "progenitor T cells (proT)" refer to hematopoietic cells produced in the process of differentiation from hematopoietic stem cells into CD3-positive T cells in a human living body, and are positive to CD34 and CD7 (CD34⁺/CD7⁺ cells). Further, in the present specification, proT can be negative to CD43, CD1a and/or CD116.

In the present specification, "mesodermal progenitor cells" refer to, for example, cells that express at least one marker gene selected from the group consisting of T (synonymous with Brachyury), KDR, FOXF1, FLK1, BMP4, MOX1, and SDF1. Mesodermal progenitor cells are not distinguished from mesodermal cells. Cells with weak expression of the above marker genes may be referred to as "mesodermal progenitor cells."

In the present specification, "regulatory T cells" refer to T cells that have the ability to inhibit the activation of effector T cells when stimulated via T cell receptors and that are responsible for the suppression of immune responses (immune tolerance). Regulatory T cells are generally CD25⁺/FOXP3⁺ cells, and CD4⁺ or CD8⁺ cells are present therein. In the present invention, the regulatory T cells may be CD4⁺ cells (i.e., CD4⁺/CD25⁺/FOXP3⁺) or CD8⁺ cells (i.e., CD8⁺/CD25⁺/FOXP3⁺), and more preferably CD4⁺ cells. Further, the transcription factor FOXP3 is known as a master regulator of CD4⁺/CD25⁺ regulatory T cells.

In the present invention, hematopoietic stem cells, hemogenic endothelial cells, progenitor T cells, and mesodermal progenitor cells may be cells isolated from biological tissues, such as bone marrow, umbilical cord blood, and blood, or may be cells derived from pluripotent stem cells, such as ES cells and iPS cells.

In the present specification, "CD4CD8 double-positive T cells" refer to, among T cells, those with surface antigens CD4 and CD8 that are both positive (CD8⁺/CD4⁺). Since T cells can be identified by surface antigens CD3 and CD45 being positive, CD4CD8 double-positive T cells can be identified as cells that are positive to CD4, CD8, CD3, and CD45.

In the present specification, "CD4CD8 double-negative T cells" refer to, among T cells, those with surface antigens CD4 and CD8 that are both negative (CD8⁻/CD4⁻). Since T cells can be identified by surface antigens CD3 and CD45 being positive, CD4CD8 double-negative T cells can be identified as cells that are negative to CD4 and CD8, and positive to CD3 and CD45.

The various cells used in the present invention are preferably cells certified by the Good Manufacturing Practice (GMP), in terms of therapeutic applications.

Examples of pluripotent stem cells include embryonic stem cells (ES cells), induced pluripotent stem cells (iPS cells), embryonal carcinoma cells (EC cells), embryonic germ stem cells (EG cells), and Muse cells; and preferably iPS cells (more preferably human iPS cells). When the pluripotent stem cells are ES cells or any cells derived from the human embryo, the cells may be produced by destroying the embryo or without destroying the embryo, and preferably cells produced without destroying the embryo.

Regarding "ES cells," various mouse ES cell strains established by Ingenious Targeting Laboratory, RIKEN, etc. can be used as mouse ES cells, and various human ES cell strains established by the University of Wisconsin, NIH, RIKEN, Kyoto University, National Center for Child Health and Development, Cellartis, etc. can be used as human ES cells. Usable examples of human ES cell strains include CHB-1 to CHB-12 strains, RUES1 strain, RUES2 strain, HUES1 to HUES28 strains, etc. furnished by ESI Bio; H1 strain, H9 strain, etc. furnished by WiCell Research; and KhES-1 strain, KhES-2 strain, KhES-3 strain, KhES-4 strain, KhES-5 strain, SSES1 strain, SSES2 strain, SSES3 strain, etc. furnished by RIKEN.

"Induced pluripotent stem cells" refer to cells obtained by reprogramming mammalian somatic cells or undifferentiated stem cells by introduction with specific factors (nuclear reprogramming factors). Currently, there are many different types of induced pluripotent stem cells. Usable examples include iPS cells established by Yamanaka et al. by introducing four factors, Oct3/4, Sox2, Klf4, and c-Myc, into mouse fibroblasts (Takahashi K., Yamanaka S., Cell, (2006) 126: 663-676), as well as human cell-derived iPS cells established by introducing the same four factors into human fibroblasts (Takahashi K., Yamanaka S., et al. Cell, (2007) 131: 861-872), Nanog-iPS cells established by introducing the above four factors, followed by screening using the expression of Nanog as an index (Okita, K., Ichisaka, T., and Yamanaka, S. (2007). Nature 448, 313-317), iPS cells produced by a method that does not include c-Myc (Nakagawa M., Yamanaka S., et al. Nature Biotechnology, (2008) 26, 101-106), and iPS cells established by introducing six factors by a virus-free method (Okita K. et al. Nat. Methods 2011 May; 8(5): 409-12, Okita K. et al. Stem Cells. 31(3): 458-66). Other usable examples include induced pluripotent stem cells produced by Thomson et al. and established by introducing four factors, OCT3/4, SOX2, NANOG, and LIN28 (Yu J., Thomson J.A. et al., Science (2007) 318: 1917-1920), induced pluripotent stem cells produced by Daley et al. (Park I.H., Daley G.Q. et al., Nature (2007) 451: 141-146), induced pluripotent stem cells produced by Sakurada et al. (JP2008-307007A), and the like.

Other usable examples are any of the induced pluripotent stem cells known in the art and disclosed in all of the published articles (e.g., Shi Y., Ding S., et al., Cell Stem Cell, (2008) Vol. 3, Issue 5, 568-574; Kim J.B., Scholer H.R., et al., Nature, (2008) 454, 646-650; Huangfu D., Melton, D.A., et al., Nature Biotechnology, (2008) 26, No. 7, 795-797), or patents (e.g., JP2008-307007A, JP2008-283972A, US2008/2336610, US2009/047263, WO2007/069666, WO2008/118220, WO2008/124133, WO2008/151058, WO2009/006930, WO2009/006997, and WO2009/007852).

As induced pluripotent stem cell strains, various iPS cell strains established by NIH, RIKEN, Kyoto University, etc. can be used. Examples of human iPS cell strains include HiPS-RIKEN-1A strain, HiPS-RIKEN-2A strain, HiPS-RIKEN-12A strain, and Nips-B2 strain (RIKEN); Ff-I01s04 strain, QHJI strain, RWMH strain, DRXT strain, RJWI strain, YZWJ strain, ILCL strain, GLKV strain, 253G1 strain, 201B7 strain, 409B2 strain, 454E2 strain, 606A1 strain, 610B1 strain, and 648A1 strain (Kyoto University); and the like.

The "nucleic acid" may be any molecule obtained by polymerizing a nucleotide and a molecule having the same function as the nucleotide. Examples include RNA that is a polymer of ribonucleotide, DNA that is a polymer of deoxyribonucleotide, a polymer that is a mixture of ribonucleotide and deoxyribonucleotide, and a nucleotide polymer containing a nucleotide analog. The nucleic acid may also be a nucleotide polymer containing a nucleic acid derivative. The nucleic acid may be a single-stranded nucleic acid or a double-stranded nucleic acid. Double-stranded nucleic acids include a double-stranded nucleic acid in which one strand hybridizes to the other strand under stringent conditions.

The nucleotide analog may be any molecule as long as it is a molecule obtained by modifying ribonucleotide, deoxyribonucleotide, RNA, or DNA, for improvement of nuclease resistance, stabilization, increase in affinity with complementary strand nucleic acids, enhancement of cell permeability, or visualization, compared with RNA or DNA. The nucleotide analog may be a naturally occurring molecule or a non-natural molecule. Examples include sugar-modified nucleotide analogs (e.g., 2'-O-methylribose-substituted nucleotide analog, 2'-O-propylribose-substituted nucleotide analog, 2'-methoxyethoxyribose-substituted nucleotide analog, 2'-O-methoxyethylribose-substituted nucleotide analog, 2'-O-[2-(guanidium)ethyl]ribose-substituted nucleotide analog, 2'-fluororibose-substituted nucleotide analog, bridged nucleic acid (BNA), locked nucleic acid (LNA), ethylene-bridged nucleic acid (ENA), peptide nucleic acid (PNA), oxy-peptide nucleic acid (OPNA), and peptide ribonucleic acid (PRNA)), phosphodiester bond-modified nucleotide analogs (e.g., phosphorothioate bond-substituted nucleotide analog and N3'-P5' phosphoramidate bond-substituted nucleotide analog), and the like.

The nucleic acid derivative may be any molecule as long as it is a molecule obtained by adding another chemical substance to a nucleic acid, for improvement of nuclease resistance, stabilization, increase in affinity with complementary strand nucleic acids, enhancement of cell permeability, or visualization, compared with the nucleic acid. Specific examples include 5'-polyamine-adduct derivatives, cholesterol-adduct derivatives, steroid-adduct derivatives, bile acid-adduct derivatives, vitamin-adduct derivatives, Cy5-adduct derivatives, Cy3-adduct derivatives, 6-FAM-adduct derivatives, biotin-adduct derivatives, and the like.

The method for producing regulatory T cells of the present invention (also referred to simply as "the production method of the present invention" in the present specification) characteristically comprises the following step:
(1) differentiating cells that can differentiate into regulatory T cells, into which an expression construct is introduced, into regulatory T cells, the expression construct (also referred to as "the expression construct of the present invention," "the expression construct," "the CNS-Foxp3 construct," or "the construct" in the present specification) comprising:
   (a) conserved non-coding sequence (CNS) 1, CNS2, and CNS3 of Foxp3 gene;
   (b) a promoter; and
   (c) a nucleic acid encoding FOXP3.

The expression construct of the present invention is for expressing FOXP3, and comprises (a) CNS1 (conserved non-coding sequence 1), CNS2 (conserved non-coding sequence 2), and CNS3 (conserved non-coding sequence 3) of Foxp3 gene, (b) a promoter, and (c) a nucleic acid encoding FOXP3. Regulatory T cells can be produced with high efficiency by introducing such an expression construct into cells that can differentiate into regulatory T cells. The expression construct is not particularly limited as long as it can express FOXP3. The expression construct preferably contains a terminator, a polyadenylation signal, Foxp3 3'UTR, etc., in addition to (a), (b), and (c) mentioned above, and is more preferably one that functions in cells into which these are introduced.

The base sequences of human Foxp3 gene are registered as RefSeq Accession Nos. NM_001114377 (SEQ ID No. 1) and NM_014009 (SEQ ID No. 2), and the amino acid sequences thereof are also registered as RefSeq Accession Nos. NP_001107849 (SEQ ID No. 3) and NP_054728 (SEQ ID No. 4). These RefSeq IDs are registered on the NCBI website. In the present invention, the above gene also includes degenerate products and variants of genes other than those having the base sequences registered in the database mentioned above. Desirable variants are those encoding proteins having a biological activity equivalent to that of the protein consisting of the above amino acid sequences. Examples of variants include FOXP3 variants having an amino acid sequence that is 80% or more, 85% or more, 90% or more, 91% or more, 92% or more, 93% or more, 94% or more, 95% or more, 96% or more, 97% or more, 98% or more, or 99% or more, identical to the natural amino acid sequence. In the present specification, FOXP3 refers to a protein, and Foxp3 refers to a gene; however, FOXP3 and Foxp3 refer to a gene and protein, respectively, when such an interpretation is appropriate.

The nucleic acid encoding FOXP3 is not particularly limited as long as it is a nucleic acid encoding FOXP3 protein, and preferably cDNA of FOXP3.

CNS1, CNS2, and CNS3 used in the present invention are all derived from Foxp3 gene. CNS1, CNS2, and CNS3 are Foxp3 enhancer elements. The promoter used in the present invention is not particularly limited, and examples include CAG promoter, ubiquitin gene promoter, Foxp3 gene promoter, EF1α promoter, SRα promoter, SV40 promoter, LTR promoter, cytomegalovirus (CMV) promoter, Rous sarcoma virus (RSV) promoter, Moloney murine leukemia virus (MoMuLV) LTR, herpes simplex virus thymidine kinase (HSV-TK) promoter, and the like; and preferably Foxp3 gene promoter. Preferred examples of the base sequences of CNS1, CNS2, CNS3, and promoter of human Foxp3 gene include the base sequences represented by SEQ ID Nos. 5 to 8, respectively. The promoter, CNS1, CNS2, and CNS3 include variants even if they do not have the base sequences described above. Desirable variants are those having a biological activity equivalent to that of the promoter, CNS1, CNS2, and CNS3 consisting of the above base sequences. Examples of variants include those having a base sequence that is 80% or more, 85% or more, 90% or more, 91% or more, 92% or more, 93% or more, 94% or more, 95% or more, 96% or more, 97% or more, 98% or more, or 99% or more, identical to the natural base sequence.

The arrangement (order) of the promoter, CNS1, CNS2, CNS3, and nucleic acid encoding FOXP3 in the expression construct is not particularly limited. It is preferable that CNS1, CNS2, and CNS3 are located upstream of the promoter; and the order of CNS1, CNS2, CNS3, promoter, and nucleic acid encoding FOXP3 from the 5'-terminal side is more preferred.

The cells that can differentiate into regulatory T cells, into which the above expression construct is introduced, are not particularly limited as long as they can differentiate into regulatory T cells. Examples include pluripotent stem cells, hematopoietic stem cells, hemogenic endothelial cells, progenitor T cells, mesodermal progenitor cells, and the like; preferably pluripotent stem cells; and more preferably iPS cells.

The cells that can differentiate into regulatory T cells may be derived from humans or mammals other than humans (non-human mammals), and preferably human-derived cells. Examples of non-human mammals include mice, rats, hamsters, guinea pigs, rabbits, dogs, cats, pigs, cows, horses, sheep, and monkeys.

The means for introducing the expression construct into the cells that can differentiate into regulatory T cells is not particularly limited, and various known or general means can be used. Typically, the expression construct is introduced into the cells that can differentiate into regulatory T cells using an expression vector, and is expressed. The expression vector may be linear or cyclic, and may be a non-viral vector such as a plasmid, a viral vector, or a transposon vector.

The means for introducing the expression vector into the cells that can differentiate into regulatory T cells can be made appropriate according to the embodiment. For example, the expression vector can be introduced into the cells that can differentiate into regulatory T cells by a known method, such as a virus infection method, a calcium phosphate method, a lipofection method, a microinjection method, or an electroporation method. The expression vector can be prepared in a form suitable for use in each method by known means, or using commercially available kits as appropriate (according to the instructions thereof).

The expression vector can be introduced into the cells that can differentiate into regulatory T cells by a virus infection method. Examples of viral vectors include retroviral vectors, lentiviral vectors, adenoviral vectors, and adeno-associated viral vectors. When using these viral vectors, a vector containing the expression construct and a packaging vector (plasmid) of each virus may be transfected into host cells using a corresponding commercially available kit to produce a recombinant virus, and then the cells that can differentiate into regulatory T cells may be infected with the obtained recombinant virus.

In addition to the expression construct, the expression vector may contain sequences, such as nuclear localization signal (NLS) and multi-cloning site (MCS), if necessary. The expression vector may further contain a nucleic acid (base sequence) encoding "functional genes," such as reporter genes (e.g., genes encoding various color fluorescent proteins), drug selection genes (e.g., kanamycin resistance gene, ampicillin resistance gene, and puromycin resistance gene), and suicide genes (e.g., genes encoding diphtheria A toxin, herpes simplex virus thymidine kinase (HSV-TK), carboxypeptidase G2 (CPG2), carboxylesterase (CA), cytosine deaminase (CD), cytochrome P450 (cyt-450), deoxycytidine kinase (dCK), nitroreductase (NR), purine nucleoside phosphorylase (PNP), thymidine phosphorylase (TP), varicella zoster virus thymidine kinase (VZV-TK), xanthine-guanine phosphoribosyltransferase (XGPRT), inducible caspase 9, etc.).

The production method of the present invention may further comprise (2) introducing an expression construct into cells that can differentiate into regulatory T cells, the expression construct comprising:
(a) CNS1, CNS2, and CNS3 of Foxp3 gene;
(b) a promoter; and
(c) a nucleic acid encoding FOXP3.

Examples of the method for introducing the expression construct into the cells that can differentiate into regulatory T cells include those mentioned above.

The differentiation of the cells that can differentiate into regulatory T cells, into which the expression construct is introduced, into regulatory T cells can be induced according to a known method. Examples of such methods include the artificial thymic organoid (ATO) method (see WO2017/075389 etc.). The ATO method comprises culturing a three-dimensional cell aggregate containing cells that can differentiate into regulatory T cells or cells differentiated therefrom, and stromal cells expressing a Notch ligand, whereby differentiation into regulatory T cells can be induced with high efficiency. The ATO method is a known method, and can be carried out with reference to the disclosure of WO2017/075389 etc. Other methods include the differentiation induction method (e.g., co-culture with MS5-DLL1/4 stromal cells, OP9 or OP9-DLL1 stromal cells, or EpCAM-CD56⁺ stromal cells) disclosed in WO2021/092581. The stromal cells expressing a Notch ligand are preferably mouse-derived stromal cell strain MS5 in which human DLL4 protein is forcibly expressed.

The cells that can differentiate into regulatory T cells or cells differentiated therefrom refer to "cells that can differentiate into regulatory T cells, into which the expression construct is introduced" or "cells differentiated from the cells that can differentiate into regulatory T cells, into which the expression construct is introduced." The ATO method may be performed on the cells that can differentiate into regulatory T cells, into which the expression construct is introduced (i.e., the cells into which the expression construct is to be introduced), or on cells differentiated from the cells that can differentiate into regulatory T cells, into which the expression construct is introduced (i.e., the cells into which the expression construct is to be introduced).

The cells that can differentiate into regulatory T cells or cells differentiated therefrom, on which the ATO method is performed, are not particularly limited. Examples include CD34⁺ cells, mesodermal progenitor cells, CD4CD8 double-negative T cells, CD4CD8 double-positive T cells, and the like; preferably CD34⁺ cells or mesodermal progenitor cells; more preferably CD34⁺/CD7⁻ cells or mesodermal progenitor cells; even more preferably hemogenic endothelial cells, hematopoietic stem cells, and mesodermal progenitor cells; and particularly preferably hemogenic endothelial cells. The cells to be subjected to the ATO method are more desirably cells differentiated from the cells that can differentiate into regulatory T cells. The CD34⁺ cells are cells that express CD34 (CD34⁺), and particularly cells that express CD34 but do not express CD7 (CD34⁺/CD7⁻). Examples of the CD34⁺ cells include hemogenic endothelial cells and hematopoietic stem cells.

In particular, it is desirable to use pluripotent stem cells (in particular, iPS cells) as the cells that can differentiate into regulatory T cells, induce the differentiation of the cells into CD34⁺ cells (in particular, hemogenic endothelial cells), and subject the CD34⁺ cells (in particular, hemogenic endothelial cells) to the ATO method for differentiation into regulatory T cells.

The differentiation of the pluripotent stem cells into CD34⁺ cells can be induced according to a known method. When the pluripotent stem cells are iPS cells, the differentiation of hematopoietic progenitor cells can be induced, for example, by the methods disclosed in WO2017/221975, WO2018/135646, and Cell Reports 2(2012)1722-1735, thereby producing CD34⁺ cells. When hemogenic endothelial cells are subjected to the ATO method, the ATO method can be performed on CD34⁺ cells containing hemogenic endothelial cells (i.e., not separating hemogenic endothelial cells before performing the ATO method), or the ATO method can also be performed on hemogenic endothelial cells separated according to a known method (e.g., flow cytometry or a magnetic cell separation method).

The Notch ligand is not particularly limited, and includes the canonical Notch ligand and non-canonical Notch ligand disclosed in WO2017/075389. Examples of the canonical Notch ligand include DLL4 (Delta-like ligand 4), DLL1 (Delta-like ligand 1), JAG1 (Jagged 1), JAG2 (Jagged 2), and the like. These can be used singly or in combination of two or more. Examples of the non-canonical Notch ligand include Contactin-1, NOV/CCN3, Contactin-6, Periostin/OSF-2, DLK2/EGFL9, Pref-1/DLK1/FA1, DNER, Thrombospondin-2, MAGP-1/MFAP2, Thrombospondin-3, MAGP-2/MFAP5, Thrombospondin-4, and Netrin-1. Human DLL4 is preferably used as the Notch ligand.

The three-dimensional cell aggregate can be formed, for example, by centrifuging cells that can differentiate into regulatory T cells or cells differentiated therefrom, and stromal cells expressing a Notch ligand. Examples of stromal cells include mouse stromal cell strains, human stromal cell strains, and the like. It is preferable that a nucleic acid for expressing the Notch ligand is introduced into the stromal cells. The ratio of the stromal cells to the cells that can differentiate into regulatory T cells or cells differentiated therefrom is, for example, 100:1 to 1:100, 20:1 to 1:20, or 10:1 to 4:1, and preferably 10:1 to 4:1.

The medium for culturing the three-dimensional cell aggregate is not particularly limited, and examples include a serum-free medium, and particularly a serum-free medium containing insulin (further biotin, transferrin, and albumin).

Examples of the basal medium for culturing the three-dimensional cell aggregate include AIMV, X-VIVO-15, NeuroBasal, EGM2, TeSR, BME, BGJb, CMRL1066, Glasgow's MEM, Improved MEM Zinc Option, IMDM, 199 medium, Eagle's MEM, aMEM, DMEM, Ham, RPMI-1640, Fischer's medium, and the like. These media may be used singly or as a mixture of two or more. In addition, for example, the medium components disclosed in WO2017/075389 may be suitably added.

As the culture conditions for culturing the three-dimensional cell aggregate, for example, the culture temperature is about 20 to 40°C, the CO₂ concentration is about 2 to 10%, the oxygen concentration is about 1 to 20%, and the culture period is, for example, about 1 to 100 days. The cell density at the start of culture can be, for example, about 1.0×10⁴ to 1.0×10¹⁰ cells/mL.

The medium, culture conditions, and the like used in the production method of the present invention can be those suitable for the type of cell to be cultured.

The basal medium used as such a medium is not particularly limited as long as it can be used to culture animal cells. Examples include those mentioned above.

The medium may contain serum or may be serum-free. The medium may further contain serum replacements (e.g., albumin, transferrin, Knockout Serum Replacement (KSR), fatty acid, insulin, collagen precursor, minor element, 2-mercaptoethanol, 3'-thiolglycerol, and ITS-supplement). Serum replacements can be used singly or in combination of two or more.

The medium may further contain one or more substances of lipids, amino acids (non-essential amino acids etc.), L-glutamine, vitamins, growth factors, cytokines, antibiotics, antioxidants, pyruvic acid, buffers, inorganic salts, etc. As the medium, it is desirable to use a chemically-defined medium that does not contain materials with unknown components, such as serum, because the difference in medium among lots can be reduced, and cells with stable quality can be prepared.

The pH of the medium is generally 7.0 to 7.8, and preferably 7.2 to 7.6. In order to prevent contamination before use, the medium is preferably sterilized by a method such as filtration, UV irradiation, heat sterilization, or radiation. The culture is carried out in the presence or absence of feeder cells. The culture conditions are not particularly limited, and conditions generally used for cell culture can be used. In the culture, passage may be performed as many times as necessary to obtain the desired amount of cells, or addition and replacement of medium may be performed. The culture container is not particularly limited, and can be suitably selected from plates, dishes, petri dishes, flasks, bags, bottles, tanks (culture tanks), bioreactors, and the like.

The production method of the present invention may further comprise separating the obtained regulatory T cells to concentrate the regulatory T cells. The regulatory T cells can be separated by a known method, such as a method using flow cytometry, or a magnetic cell separation method.

The production method of the present invention may further comprise:
(3A) culturing the regulatory T cells in the presence of at least one substance selected from the group consisting of CDK8 and/or CDK19 inhibitors, TNFR2 agonists, mTOR inhibitors, and TGF-βR agonists.

The production method of the present invention may preferably further comprise:
(3) culturing the regulatory T cells in the presence of an mTOR inhibitor and a TNFR2 agonist.

CDK8 (cyclin-dependent kinase 8) and/or CDK19 inhibitors are defined as substances that inhibit the function of CDK8 and/or CDK19, and particularly substances that inhibit the kinase activity of CDK8 and/or CDK19. They may be inhibitors for both or one of CDK8 and CDK19. Examples of CDK8 and/or CDK19 inhibitors include 4-[1-(2-methyl-1H-benzimidazol-5-yl)-1H-imidazo[4,5-c]pyridin-2-yl]-1,2,5-oxadiazol-3-amine (AS2863619) (hereinafter also referred to as "compound 1"); 3-{1-[1-(4-methoxyphenyl)piperidin-4-yl]-4-methyl-1H-imidazo[4,5-c]pyridin-2-yl}pyrazin-2-amine (AS3334366) (hereinafter also referred to as "compound 2"); siRNA, shRNA, dsRNA, miRNA, and antisense nucleic acid for genes encoding CDK8 and/or CDK19 and/or transcripts thereof, and expression vectors expressing them; and the compounds disclosed in US8598344, WO2013/116786, Proc. Natl. Acad. Sci. U.S.A. 109 13799-13804 (2012), WO2013/001310, WO2013/040153, WO2014/029726, WO2014063778, WO2014/072435, WO2014/090692, WO2014/106606, WO2014/123900, WO2014/154723, WO2014/194245, WO2015/049325, WO2015/100420, WO2015/144290, WO2015/159937, WO2015/159938, WO2016/009076, and WO2018/159805. Preferred among these are compound 1, compound 2, and siRNA for genes encoding CDK8 and/or CDK19 and/or transcripts thereof; and more preferred is compound 1. The CDK8 and/or CDK19 inhibitors can be used singly or in combination of two or more.

mTOR (mechanistic target of rapamycin) inhibitors are defined as substances that inhibit the function of mTOR, and these include substances that inhibit the function of mTOR itself, and substances that inhibit the function of mTOR complex 1 (mTORC1) and mTOR complex 2 (mTORC2), which are mTOR complexes. Examples of mTOR inhibitors include rapamycin or derivatives thereof, everolimus, temsirolimus, ridaforolimus, sirolimus, KU0063794, AZD805, AZD8055, WYE-354, WAY-600, WYE-687, Pp121, Pp242, Dactolisib, Sapanisertib, Omipalisib, Vistusertib, Torin 1, Torin 2, and the like. Other examples of mTOR inhibitors include siRNA, shRNA, dsRNA, miRNA, and antisense nucleic acid for genes encoding mTOR and/or transcripts thereof, and expression vectors expressing them; and further include siRNA, shRNA, dsRNA, miRNA, and antisense nucleic acid for genes encoding enzymes that phosphorylate and activate mTOR and/or transcripts thereof, and expression vectors expressing them. Of these, the mTOR inhibitor is preferably rapamycin or a derivative thereof, and more preferably rapamycin. The mTOR inhibitors can be used singly or in combination of two or more.

TNFR2 (tumor necrosis factor receptor-2) agonists are defined as substances that can bind to TNFR2 to activate TNFR2 signaling. Examples include antibodies (e.g., anti-TNFR2 antibody), peptides, low-molecular-weight compounds, proteins, and the like. Examples of TNFR2 agonist antibodies include monoclonal antibodies binding to TNFR2, such as clone MR2-1 (Hycult Biotech) and clone MAB2261 (R&D Systems). The TNFR2 agonist may be TNF-α mutein that binds to only TNFR2 as an agonist. The TNFR2 agonists can be used singly or in combination of two or more.

The TNFR2 agonist is preferably TNFR2 agonist antibody. The antibody may be a functional fragment thereof. Examples of functional fragments include Fd, Fv, Fab, F(ab'), F(ab)₂, F(ab')₂, single-chain Fv (scFv), diabody, triabody, tetrabody, and minibody. Examples of the antibody include those derived from animals, such as mice, rats, cows, rabbits, goats, sheep, and guinea pigs. The isotype of the antibody is not particularly limited. Examples of the isotype include IgG (IgG1, IgG2, IgG3, and IgG4), IgA, IgD, IgE, and IgM. The antibody may be a monoclonal antibody or a polyclonal antibody, and preferably a monoclonal antibody. Further, the antibody may be a humanized antibody, a chimeric antibody, a multispecific antibody (e.g., a bispecific antibody), or the like. The antibody can be produced by a known method. For example, the antibody can be produced by constructing an expression vector containing a nucleic acid encoding the antibody, and culturing a transformant into which the nucleic acid is introduced, or culturing a hybridoma that produces the antibody.

TGF-βK (transforming growth factor-β receptor) agonists are defined as substances that can bind to TGF-βR to activate TGF-βR signaling. Examples of TGF-βR agonists include factors belonging to the TGF-β superfamily. The TGF-β superfamily includes TGF-β family, activin family, and BMP (bone morphogenetic protein) family. Examples of factors belonging to the TGF-β superfamily include TGF-β (TGF-β1, TGF-β2, and TGF-β3), activin A, activin B, GDF-8, GDF-11, and the like. The TGF-βR agonist is preferably TGF-β. The TGF-βR agonists can be used singly or in combination of two or more.

Further, the CDK8 and/or CDK19 inhibitor, TNFR2 agonist, mTOR inhibitor, and TGF-βR agonist can be used in free form or in salt form. Examples of salts include salts with inorganic bases, such as sodium salts, magnesium salts, potassium salts, calcium salts, and aluminum salts; salts with organic bases, such as methylamine salts, ethylamine salts, and ethanolamine salts; salts with basic amino acids, such as lysine, ornithine, and arginine; and ammonium salts. These salts may be acid addition salts. Specific examples of such salts include acid addition salts with mineral acids, such as hydrochloric acid, hydrobromic acid, hydroiodic acid, sulfuric acid, nitric acid, and phosphoric acid; organic acids, such as formic acid, acetic acid, propionic acid, oxalic acid, malonic acid, malic acid, tartaric acid, fumaric acid, succinic acid, lactic acid, maleic acid, citric acid, methanesulfonic acid, and ethanesulfonic acid; and acidic amino acids, such as aspartic acid and glutamic acid. The CDK8 and/or CDK19 inhibitor, TNFR2 agonist, mTOR inhibitor, and TGF-βR agonist also include hydrates, solvates, polymorphs, and the like.

When the CDK8 and/or CDK19 inhibitor, TNFR2 agonist, mTOR inhibitor, and TGF-βR agonist contain asymmetric carbon, these include R-isomers, S-isomers, and mixtures containing both isomers in any ratio (e.g., racemates).

The concentration of the CDK8 and/or CDK19 inhibitor in the medium is not particularly limited as long as the regulatory T cells can be expansively cultured, and is suitably adjusted according to the type of CDK8 and/or CDK19 inhibitor used, etc. The concentration of the CDK8 and/or CDK19 inhibitor is, for example, 0.1 to 300 nM, and preferably 0.5 to 150 nM.

The concentration of the mTOR inhibitor in the medium is not particularly limited as long as the regulatory T cells can be expansively cultured, and is suitably adjusted according to the type of mTOR inhibitor used, etc. The concentration of the mTOR inhibitor is, for example, 1 to 3000 nM, preferably 5 to 1000 nM, and more preferably 10 to 100 nM.

The concentration of the TNFR2 agonist in the medium is not particularly limited as long as the regulatory T cells can be expansively cultured, and is suitably adjusted according to the type of TNFR2 agonist used, etc. The concentration of the TNFR2 agonist is, for example, 0.0001 to 100 µg/mL, preferably 0.01 to 10 µg/mL, and more preferably 1 to 5 µg/mL.

The concentration of the TGF-βR agonist in the medium is not particularly limited as long as the regulatory T cells can be expansively cultured, and is suitably adjusted according to the type of TGF-βR agonist used, etc. The concentration of the TGF-βR agonist is, for example, 0.1 to 100 ng/mL, and preferably 1 to 50 ng/mL.

The medium used in the culture of step (3) or (3A) uses a medium used for culturing animal cells as the basal medium, and contains the CDK8 and/or CDK19 inhibitor, TNFR2 agonist, mTOR inhibitor, and TGF-βR agonist described above. The medium and culture conditions include those mentioned above. The culture period is also not particularly limited, and can be suitably determined by a person skilled in the art while monitoring the number of regulatory T cells. The culture period is, for example, 10 days or more, preferably 1 week or more, and more preferably 2 weeks or more. The upper limit of the culture period is not particularly limited, and is, for example, 35 days or less, preferably 28 days or less, and more preferably 21 days or less.

A foreign gene may be introduced into the regulatory T cells obtained by the production method of the present invention to further produce regulatory T cells into which the foreign gene is introduced.

The "foreign gene" is a gene to be introduced from the outside in order to express the desired protein in the regulatory T cells, and can be suitably selected depending on the use of the regulatory T cells.

The foreign gene can be, for example, a gene for expressing a chimeric antigen receptor (CAR), and can further contain a gene for expressing a cytokine and/or a chemokine. As with general or known CARs, the CARs expressed by the regulatory T cells are basically configured such that peptides at sites of (i) an antigen recognition site that recognizes cell surface antigens of cancer cells (e.g., single-chain antibody), (ii) a transmembrane region, and (iii) a signal transduction region that induces the activation of T cells, are linked via a spacer, as needed. The foreign gene can also be, for example, a gene for expressing an exogenous T-cell receptor (TCR). The exogenous TCR means that it is exogenous to T cells into which a nucleic acid encoding the exogenous TCR is to be introduced. The amino acid sequence of the exogenous TCR may be identical to or different from that of the endogenous TCR of the T cells. One or two or more foreign genes may be introduced (e.g., CAR and exogenous TCR).

Examples of the means for introducing the foreign gene into the regulatory T cells include the methods mentioned above.

The regulatory T cells produced by the production method of the present invention are useful for the treatment of animals (in particular, humans) with an abnormally enhanced immune response. For example, the regulatory T cells are useful for the treatment and prevention of immune dysregulation polyendocrinopathy enteropathy X-linked (IPEX) syndrome, graft-versus-host disease (GVHD), rejection in organ transplantation, autoimmune diseases, inflammatory diseases, allergic diseases (hay fever, asthma, atopic dermatitis, eczema, food allergy, food hypersensitivity, urticaria, allergic rhinitis, allergic conjunctivitis, and drug allergy), and the like, but are not limited thereto. The regulatory T cells produced by the production method of the present invention may be used for autologous transplantation or allogeneic transplantation. Further, the regulatory T cells may be used in combination with other drugs.

When performing such cell therapy, from the viewpoint that rejection does not occur, the subject from which cells are isolated for use in the production of regulatory T cells preferably has the same HLA type as a subject to which regulatory T cells are to be administered, and is more preferably the same as the subject to which regulatory T cells are to be administered.

The present invention can produce a pharmaceutical composition comprising regulatory T cells (hereinafter also referred to as "the pharmaceutical composition of the present invention"). The pharmaceutical composition of the present invention is preferably produced as a parenteral preparation by mixing an effective amount of regulatory T cells with a pharmaceutically acceptable carrier according to known means (e.g., the methods described in the Japanese Pharmacopoeia). The pharmaceutical composition of the present invention is preferably produced as a parenteral preparation, such as injection, suspension, or infusion. Examples of parenteral administration methods include intravenous, intraarterial, intramuscular, intraperitoneal, or subcutaneous administration. Examples of the pharmaceutically acceptable carrier include solvents, bases, diluents, excipients, soothing agents, buffers, preservatives, stabilizers, suspensions, isotonic agents, surfactants, solubilizing agents, and the like.

The dose of the pharmaceutical composition of the present invention can be suitably determined depending on various conditions, such as patient's body weight, age, sex, and symptoms. In general, the pharmaceutical composition of the present invention is administered so that the number of cells per administration for a subject with a body weight of 60 kg is generally 1×10⁶ to 1×10¹⁰ cells, preferably 1×10⁷ to 1×10⁹ cells, and more preferably 5×10⁷ to 5×10⁸ cells. The pharmaceutical composition of the present invention may be administered once or several times. The pharmaceutical composition of the present invention can have a known form suitable for parenteral administration, such as injection or infusion. Further, the pharmaceutical composition of the present invention may contain physiological saline, phosphate buffered saline (PBS), medium, and the like in order to stably maintain the cells. Examples of the medium include RPMI, AIM-V, X-VIVO10, and other media, but are not limited thereto. In addition, pharmaceutically acceptable carriers (e.g., human serum albumin), preservatives, and the like may be added to the pharmaceutical composition for the purpose of stabilization. The pharmaceutical composition of the present invention is applied to mammals, including humans.

As used in the present specification and claims, unless otherwise required by context, singular terms shall include pluralities and plural terms shall include the singular. Thus, singular articles (e.g., "a," "an," "the," and the like in the case of English) should also be understood as encompassing the concepts thereof in the plural form unless specifically noted otherwise.

### Examples

Examples are provided below in order to explain the present invention in more detail. However, the present invention is not limited to these Examples.

### (1) Production of CNS-Foxp3 Construct

A plasmid sequence was designed and synthesized by introducing, into a transfer plasmid for producing a third-generation lentiviral vector, a DNA sequence obtained by changing mStrawberry protein sequence in the sequence (MK012431) registered in GenBank to dTomato sequence. The received plasmid was transfected into HEK293 lineage cells together with a packaging plasmid and an envelope plasmid for producing a lentiviral vector. The supernatant containing the produced lentiviral vector was collected, and then concentrated by a high-speed centrifugation method to obtain a lentiviral vector to be introduced into iPS cells.

### (2) Introduction of Construct into iPS Cells and Differentiation of Construct-Introduced iPS Cells into HEC

The cell population containing hemogenic endothelial cells used was a floating cell population differentiated from iPS cells (Ff-I01s04 strain: derived from healthy peripheral blood mononuclear cells) provided by the Center for iPS Cell Research and Application, Kyoto University, by known methods (e.g., the methods disclosed in Cell Reports 2(2012)1722-1735 and WO2017/221975).

Specifically, the Ff-I01s04 strain was seeded in a 24-well plate at 1×10⁴ cells/well, and protamine was added at a final concentration of 10 µg/mL. Then, a lentivirus solution with CNS-Foxp3 gene introduced was directly added to produce virally infected iPS cells. The virally infected Ff-I01s04 strain was seeded in a 6-well plate (Corning), which had been subjected to ultra-low adhesion treatment, at 1×10⁶ cells/well (Day 0). 50 ng/ml BMP4 (R&D systems), 50 ng/ml bFGF (FUJIFILM Wako Pure Chemical Corporation), 50 ng/ml VEGF (R&D systems), and 2 µM SB431542 (FUJIFILM Wako Pure Chemical Corporation) were added to EB medium (StemPro34 (Gibco) supplemented with 10 ug/ml human insulin, 5.5 ug/ml human transferrin, 5 ng/ml sodium selenite (ITS, Gibco), 2 mM L-glutamine (Sigma-Aldrich), 45 mM α-monothioglycerol (Nacalai Tesque, Inc.), and 50 µg/ml L-ascorbic acid 2-phosphate (Sigma-Aldrich)), and the cells were cultured under low oxygen conditions (5% O₂) for 4 days (Day 4). Subsequently, the cells were further cultured for 4 days in a medium supplemented with 50 ng/ml bFGF, 50 ng/ml VEGF, and 50 ng/ml SCF (R&D systems) (Day 8), thereby obtaining a cell population containing HEC. The floating cell population containing HEC was stained using an antibody set in Table 1 below.

**Table 1**

| Antibody | Vendor | Fluorescent label |
|---|---|---|
| Anti-CD34 antibody | Abcam | PE/Cy7 |
| Anti-CD43 antibody | BD | BV510 |
| Anti-CD73 antibody | BD | APC |
| Anti-CD184 antibody | BD | BV421 |
| Anti-CD235a antibody | BioLegend | FITC |
| Anti-CD14 antibody | BioLegend | APC/eF780 |

It was confirmed by a flow cytometry method that the obtained cell population contained about 15% of HEC (CD34⁺/CD43⁻ /CD73⁻/CD184⁻ cells). As a control, a cell population containing HEC (Untransduced) was obtained in the same manner as described above, except for using iPS cells (Ff-I01s04 strain) into which the expression construct produced in (1) was not introduced.

### (3) Induction of Differentiation from HEC into Treg

The cell population containing HEC obtained in (2) above was allowed to differentiate into Treg (CD25⁺/FOXP3⁺) with reference to the ATO method (the method disclosed in WO2017/075389 etc.).

Specifically, mouse-derived stromal cell strain MS5 in which human DLL 4 protein was forcibly expressed was used as a support, and co-cultured with the CNS-Foxp3-introduced human iPS cell-derived HEC produced in (2) above at a cell ratio of 4:1. The medium used was RPMI-1640 (FUJIFILM Wako Pure Chemical Corporation) containing, at a final concentration, 2x B27 supplement (Invitrogen), 1x PSG (Sigma-Aldrich), 1x Glutamax (Invitrogen), 5 ng/mL IL-7 (PeproTech), 5 ng/mL FlT3L (PeproTech), and 50 ug/mL ascorbic acid (Sigma-Aldrich). The cells co-cultured on 30 mm Millicell (hydrophilic PTFE, pore size: 0.4 µm, height: 5 mm, Merck Millipore) were allowed to stand in a 6-well plate (TPP), and cultured for 9 weeks while changing the medium once every three or four days.

After the culture, the obtained cells were collected, and using dTomato combined with the downstream of the Foxp3 gene shown in (1) above as an index, a target cell fraction was obtained using FACS Aria. This was expanded according to the method disclosed in WO2020/032179 to ensure a sufficient number of cells, and then intracellularly stained using an antibody set in Table 2 below, thereby assessing the induction of human iPS cell-derived Treg. It was confirmed with a flow cytometer (FACS Aria Fusion, BD Bioscience) that CD4⁺ Treg (CD4⁺/CD25⁺/FOXP3⁺) was induced. It was also confirmed that compared with the control (Untransduced), Treg was induced at a higher ratio in the CNS-Foxp3 construct-transduced group (the ratio in the Foxp3-transduced iPS cells was 56.4%, whereas it was 11.2% in the untransduced cells) (Fig. 1).

**Table 2**

| Antibody | Vendor | Fluorescent label |
|---|---|---|
| Anti-FOXP3 antibody | Abcam | FITC |
| Anti-CD25 antibody | BD | APC |
| Anti-CD4 antibody | BioLegend | BV421 |
| Anti-CD8β antibody | eBioscience | PE-Cy7 |

### (4) Expansion Culture of iPS-Treg

The cells after differentiation from iPS cells into Treg obtained in (3) above were expansively cultured in the following medium containing 10 nM of rapamycin (Merck) and 3 ug/mL of anti-TNFR2 antibody (Hycult Biotech, clone MR2-1).

Specifically, the CNS-Foxp3-introduced human iPS cell-derived Treg obtained in (3) above was cultured in an anti-CD3 antibody (eBioscience)-binding 48-well cell culture plate for 3 days, and then reseeded in a 24-well G-Rex cell culture plate, and culture was continued. The medium used was α-MEM (Invitrogen) containing, at a final concentration, FBS (15%, Corning), an L-Glutamine-Penicillin-Streptomycin solution (1/100, Invitrogen, Sigma-Aldrich), Insulin-Transferrin-Selenium Supplement (1/100, Invitrogen), ascorbic acid 2-phosphate (50 µg/mL, Sigma-Aldrich), IL-2 (10 ng/mL, PeproTech), IL-7 (10 ng/mL, PeproTech), IL-12 (50 ng/mL, Merck), IL-15 (10 ng/mL, PeproTech), IL-18 (50 ng/mL, MBL), IL-21 (20 ng/mL, PeproTech), and TL-1A (50 ng/mL, PeproTech). For the first three days, anti-CD28 antibody (1.5 µg/mL, BioLegend), anti-CD30 antibody (300 ng/mL, R&D systems), and a caspase inhibitor (10 µM, R&D systems) were further added to the above formulation. Culture was carried out for 9 or 10 days while exchanging the medium on the 3rd day after the start of culture and thereafter once every three or four days. Each cell population was stained with the following antibody set (Zombie NIR, BV510 CD3, BV421 CD4, PE/Cy7 CD8, APC CD25, FITC Foxp3). When the examination of demethylation amount of TSDR (5) and the evaluation of Treg suppressive function (6) were performed, cell populations obtaiened by, following the above expansion culture, purifying a CD4⁺Foxp3⁺ cell group using a FACSAria (Becton Dickinson) were used for the evaluation. In the purification, the following antibody set was used for staining (BV421 CD4, PE/Cy7 CD8, APC CD25).

Fig. 2 shows the results of flow cytometry analysis after expansion culture in the medium containing rapamycin and anti-TNFR2 antibody. It was confirmed that among the cell populations obtained by expansion culture in the medium containing rapamycin and anti-TNFR2 antibody, the CNS-Foxp3 construct-introduced iPS cell-derived cells maintained Treg at a higher ratio than the control (Untransduced) (the ratio in the CNS-Foxp3 construct-transduced group was 84.8%, whereas it was 10.4% in the untransduced group). Further, in the cell populations obtained by expansion culture in the medium not containing rapamycin and TNFR2Ab, the CNS-Foxp3 construct-introduced iPS cell-derived cells maintained Treg at a higher ratio than the control (Untransduced) (the ratio in the CNS-Foxp3 construct-transduced group was 56.4%, whereas it was 10.1% in the untransduced group).

### (5) Examination of Demethylation Amount of Treg-Specific Demethylated Region (TSDR)

Using the cells after differentiation from iPS cells into Treg, TSDR demethylation was examined. The cells used are as follows. Primary Treg: a Treg (CD3⁺/CD4⁺/CD8⁻/CD25⁺/CD127⁻) cell population isolated from human PBMC; Primary Non-Treg: a non-Treg (CD3⁺/CD4⁺/CD8⁻/CD25⁻) cell population isolated from human PBMC; iPSC-Treg: a Treg (CD3⁺/CD4⁺/CD8⁻/CD25⁺/Foxp3⁺) cell population differentiated from iPS cells; HEC: undifferentiated hematopoietic cells induced from iPS cells (CD34⁺/CD43⁻/CD73⁻/CD184⁻). As the iPSC-Treg, cells differentiated from CNS-Foxp3 construct-transduced iPS cells by the methods (1) to (3) described above were used, and as the HEC, cells differentiated by the methods (1) and (2) without transducing the CNS-Foxp3 construct were used.

Specifically, DNA was extracted from each of the above cells using a PureLink Genomic DNA Mini Kit (Thermo Fisher Scientific). For the DNA, using PureQuant Treg Assay (Thermo Fisher Scientific), demethylation of genomic DNA was quantified by qRCR. The protocol followed the recommended protocol of the kit. Fig. 3 shows the results. In iPSC-Treg, TSDR was demethylated at a higher rate than in HEC before differentiation (80% in iPSC-Treg, whereas 0% in HEC), and the rate was as high as that of Primary Treg (88% in Primary Treg).

### (6) Evaluation of Treg Suppressive Function

Using Treg differentiated from the CNS-Foxp3 construct-transduced iPS cells obtained in (3) above, analysis was conducted by flow cytometry after co-culture with human PBMC-derived allogeneic T cells.

Specifically, target cells were prepared by staining PBMC untreated or treated with anti-CD3 antibody derived from a donor different from the donor of Treg with CellTrace Violet (Thermo Fisher Scientific), and co-cultured with Treg. The medium used was α-MEM (Invitrogen) containing, at a final concentration, FBS (15%, Corning), an L-Glutamine-Penicillin-Streptomycin solution (1/100, Invitrogen, Sigma-Aldrich), Insulin-Transferrin-Selenium Supplement (1/100, Invitrogen), and ascorbic acid 2-phosphate (50 µg/mL, Sigma-Aldrich), and the cells were cultured for 4 days. Each cell population was stained with the following antibody set (Zombie NIR, PE/Cy7 CD3, FITC HLA-A24).

Fig. 4 shows the results of flow cytometry analysis after co-culture with human PBMC-derived allogeneic T cells. In the group co-cultured with iPS-Treg, cell division of the target cells was strongly suppressed compared with culture of the target cells alone (23.5% of the target cells induced cell division under the condition of Treg:Target = 2:1, whereas it was 74.9% when only the target cells were cultured). Further, its suppressive function was similar to or stronger than that of Primary Treg (when co-cultured with Primary Treg, 47.5% of the target cells induced cell division).

The present application is based on Japanese Patent Application No. 2021-157187 filed on September 27, 2021 in Japan, the entire contents of which are incorporated herein.

Sequence Listing

## Claims

1. A method for producing regulatory T cells, the method comprising:
(1) differentiating cells that can differentiate into regulatory T cells, into which an expression construct is introduced, into regulatory T cells, the expression construct comprising:
(a) conserved non-coding sequence (CNS) 1, CNS2, and CNS3 of Foxp3 gene;
(b) a promoter; and
(c) a nucleic acid encoding FOXP3.

2. The method according to claim 1, wherein the cells that can differentiate into regulatory T cells are pluripotent stem cells, hematopoietic stem cells, hemogenic endothelial cells, progenitor T cells, or mesodermal progenitor cells.

3. The method according to claim 1, wherein the cells that can differentiate into regulatory T cells are iPS cells.

4. The method according to claim 1, wherein CNS1, CNS2, and CNS3 are located upstream of the promoter.

5. The method according to claim 1, wherein the regulatory T cells are CD25⁺/FOXP3⁺ cells.

6. The method according to claim 1, further comprising:
(2) introducing an expression construct into cells that can differentiate into regulatory T cells, the expression construct comprising:
(a) CNS1, CNS2, and CNS3 of Foxp3 gene;
(b) a promoter; and
(c) a nucleic acid encoding FOXP3.

7. The method according to claim 1, wherein a three-dimensional cell aggregate is cultured in step (1), and the three-dimensional cell aggregate comprises the cells that can differentiate into regulatory T cells or cells differentiated therefrom, and stromal cells expressing a Notch ligand.

8. The method according to claim 7, wherein the cells that can differentiate into regulatory T cells or cells differentiated therefrom are CD34⁺ cells.

9. The method according to claim 7, wherein the cells that can differentiate into regulatory T cells or cells differentiated therefrom are hemogenic endothelial cells.

10. The method according to claim 7, wherein the Notch ligand is at least one member selected from the group consisting of DLL4, DLL1, JAG1, and JAG2.

11. iPS cells into which an expression construct is introduced, the expression construct comprising:
(a) CNS1, CNS2, and CNS3 of Foxp3 gene;
(b) a promoter; and
(c) a nucleic acid encoding FOXP3.

12. The iPS cells according to claim 11, wherein CNS1, CNS2, and CNS3 are located upstream of the promoter.

13. Regulatory T cells obtained by the method according to claim 1.

14. A pharmaceutical composition comprising the regulatory T cells according to claim 13.

15. The pharmaceutical composition according to claim 14, for use in prevention and/or treatment of an abnormally enhanced immune response.

16. A method for preventing and/or treating an abnormally enhanced immune response, comprising administering the regulatory T cells according to claim 13 to a subject in need thereof.

17. The regulatory T cells according to claim 13, for use in prevention and/or treatment of an abnormally enhanced immune response.

18. Use of the regulatory T cells according to claim 13 in the production of a pharmaceutical composition for use in prevention and/or treatment of an abnormally enhanced immune response.

19. The method according to claim 1, further comprising:
(3) culturing the regulatory T cells in the presence of an mTOR inhibitor and a TNFR2 agonist.
